# EUROPÄISCHE PATENTSCHRIFT

(11) **EP 2 220 367 B1**
(45) Veröffentlichungstag und Bekanntmachung des Hinweises auf die Patenterteilung: **14.12.2011**
(21) Anmeldenummer: 08851992.1
(22) Anmeldetag: 20.11.2008
(51) Int. Cl.: F03D 9/02, B01D 53/62, C01B 3/00, C07C 29/151, C10J 3/68, C25B 3/04

(54) **MODULARES, NETZUNGEBUNDENES KRAFTWERK**
MODULAR POWER PLANT UNCONNECTED TO THE GRID
CENTRALE ÉLECTRIQUE MODULAIRE, INDÉPENDANTE DU RÉSEAU

(30) Priorität: 22.11.2007 CH 18072007
(43) Veröffentlichungstag der Anmeldung: 25.08.2010
(73) Patentinhaber: SolarFuel GmbH, 70565 Stuttgart (DE)
(72) Erfinder: Gregor Waldstein, 5020 Salzburg (AT)
(74) Vertreter: Grimm, Siegfried
(86) Internationale Anmeldenummer: PCT/EP2008/009803
(87) Internationale Veröffentlichungsnummer: WO 2009/065577

(56) Entgegenhaltungen:
- WO-A-00/25380
- WO-A-2005/056737
- WO-A-2007/058608
- DE-A1- 3 933 284
- DE-A1- 4 332 789
- DE-A1- 19 802 660
- US-A- 4 085 795
- US-A- 4 627 418

## Beschreibung

Die Erfindung betrifft ein netzungebundenes Kraftwerk, welches aus CO2 der Luft, Wasser und regenerativer Energie, brennbare Kohlenwasserstoffe erzeugt und speichert, wobei dieses Kraftwerk bevorzugt auf einem Schwimmkörper installiert ist; ferner die Verwendung eines solchen Kraftwerkes und Verfahren zum Betreiben eines solchen Kraftwerkes.

Es besteht ein allgemeiner Bedarf, Energie kostengünstig und umweltneutral zur Verfügung zu stellen. Fossile Energieträger leisten derzeit den Großteil der globalen Energieversorgung. Die Verwendung dieser Energieträger ist aber durch die vorhandenen Reserven und durch die Toleranz des Weltklimas für Emissionen des Verbrennungsproduktes CO2 beschränkt. Nuklearenergie wird wegen der ungelösten Entsorgungsfrage von verantwortlichen Entscheidungsträgern abgelehnt. Ferner sind allgemein Lösungen bekannt, elektrische Energie aus erneuerbaren Energiequellen zu Verfügung zu stellen. Ferner sind Lösungen bekannt flüssige Brennstoffe aus Biomasse zu erzeugen. Biogene Treibstoffe nutzen die durch Fotosynthese aus CO2 und H2O gebildete und in Biomasse gespeicherte chemische Energie und bilden einen Teil eines geschlossenen Stoffkreislaufs. Die Nutzung von Biomasse zur Energieversorgung steht aber mit der Nutzung von Biomasse für Ernährung und andere Zwecke (Biodiversität / Lebensraum) im Konflikt. Jener Teil der Erdoberfläche, an dem für Fotosynthese geeignete Bedingungen herrschen ist klein und kaum erweiterbar. Mit heutigen (BTL) Technologien müsste 20% der globalen Fotosynthesekapazität für Energiezwecke benutzt werden. Selbst der geringe Beitrag den Biomasse heute liefert hat bereits massive unerwünschte Auswirkungen auf den Agrarmarkt. Sonnenenergie und Windenergie sind nur in geringen Energiedichten und nicht nach Bedarf verfügbar. Diese Energieformen können daher nur dann einen maßgeblichen Beitrag zur globalen Energieversorgung leisten, wenn sie großflächig "geerntet" und sinnvoll gespeichert werden können. Beide Energieformen können derzeit nur ortsfest bzw. verbunden mit dem Verbraucher ("netzgebunden") genutzt werden. Dadurch ist die Wahl der Standorte stark eingeengt. Geeignete Speichersysteme für erzeugte Energie, die eine lange Lagerung und Transport über große Entfernungen zulassen, fehlen.

Nachteilig ist bei den bekannten Verfahren ferner, dass Speichermedien für elektrische Energie eine geringe Energiedichte aufweisen; ferner können erneuerbare Energien häufig an Orten und / oder zu Zeiten günstig erzeugt werden, wo der Bedarf daran gering ist. Flüssige Energieträger hingegen, insbesondere Kohlenwasserstoffe, können leicht gelagert und transportiert werden und weisen eine hohe Energiedichte auf. Ferner besteht für diese Energieträger eine ausgebaute Infrastruktur. In diesem Zusammenhang ist Methanol als besonders vorteilhafter Energieträger zu nennen; insbesondere, da es die einfachste, bei Raumtemperatur flüssige, kohlenwasserstoffhaltige Verbindung ist. Ebenso ist Methan ein günstiger Energieträger, da für diesen KW bis zum Endverbraucher eine ausgeprägte Infrastruktur besteht.

Es sind nun bereits verschiedene Verfahren zur Herstellung von Methanol aus atmosphärischem CO2 vorgeschlagen worden.

Weimer et al, Energy Conversion Mgmt, 1996, Vol. 37, 1351 ff, diskutiert generelle Lösungsansätze zur Produktion von Methanol unter Verwendung von Solarenergie. Eines der dort allgemein und ohne konkrete Ausführung vorgestellte Verfahren umfasst die Schritte alkalische CO2 Absorption, CO2 Freisetzung, elektrolytische H2 Gewinnung und Methanolsynthese aus den Komponenten H2 und CO2. Dabei wird vorgeschlagen, solare Energie zur Deckung des benötigten Strombedarfs der gesamten Anlage heranzuziehen. Dieser Prozess, obwohl geeignet, wird als nachteilig angesehen, da Biomasse als bessere Kohlenstoffquelle zur Verfügung steht und, da die solare Stromgewinnung unter anderem unwirtschaftlich, flächenintensiv und witterungsabhängig ist. Die Problematik der nur periodisch vorhandenen elektrischen Energie wird in diesem Dokument nicht berücksichtigt.

Corbett et al, US 4339547, offenbaren einen ähnlichen Prozess zur Gewinnung von Treibstoffen (Kohlenwasserstoffen) umfassend die Schritte alkalische CO2 Absorption, CO2 Freisetzung, elektrolytische H2 Gewinnung, Methanolsynthese aus den Komponenten H2 und CO2 und Umsetzung zu Kohlenwasserstoffen mittels Fischer-Tropsch Synthese. Dieses Dokument offenbart insbesondere vorteilhafte Apparate zur CO2-Absorption.

Hardy et al, US 2005/02328833, offenbaren ebenfalls einen Prozess zur Gewinnung von Treibstoffen (Kohlenwasserstoffen) umfassend die Schritte alkalische CO2 Absorption, CO2 Freisetzung, elektrolytische H2 Gewinnung, Methanolsynthese aus den Komponenten H2 und CO2 und Umsetzung zu Kohlenwasserstoffen mittels Fischer-Tropsch Synthese. In diesem Dokument wird vorgeschlagen, die benötigte Energie durch einen Nuklearreaktor zu decken. Ein wesentlicher Zweck der gesamten dort offenbarten Anlage ist es, an Bord eines Schiffes direkt Kohlenwasserstoffe zur Verwendung als Treibstoff zu produzieren.

Borgström et al, WO2007/058608 offenbaren ein netzgebundenes System zur Erzeugung, Umwandlung und Speicherung von Energie, bei welchem überschüssige Energie zur Herstellung von Methanol aus CO2 verwendet wird und wobei dieses wieder in CO2 recycliert wird wenn Energiemangel besteht. Schröder et al, WO2005/056737, Otlewski, DE19802660 und Dodd et al, WO00/25380 offenbaren ebenfalls netzgebundene Systeme bei denen ein chemischer Energieträger erzeugt wird.

Zusammenfassend bleibt festzuhalten, dass die bekannten Prozesse in der Gesamtschau verschiedene Nachteile aufweisen. So sind die Prozesse teils unnötig kompliziert, teils wenig umweltfreundlich, und insgesamt nicht geeignet, einen Energieträger preiswert und kontinuierlich zur Verfügung zu stellen. Es ist daher eine Aufgabe der vorliegenden Erfindung, eines oder mehrere der Nachteile bekannter Anlagen zu beheben.

Die vorstehend umrissenen Aufgaben werden gemäss den unabhängigen Ansprüchen gelöst. Vorteilhafte Ausführungsformen sind in der Beschreibung und den abhängigen Ansprüchen angegeben. Weitere Aspekte der Erfindung sind in den unabhängigen Ansprüchen sowie in der Beschreibung angegeben.

Die Erfindung betrifft somit ein netzungebundenes Kraftwerk zur Erzeugung und Lagerung von brennbaren Kohlenwasserstoffen, insbesondere Methanol, Dimethylether und Methan. Die Erfindung betrifft weiter ein Verfahren zur Erzeugung von brennbaren Kohlenwasserstoffen, einen Schwimmkörper ausgerüstet mit einem Kraftwerk wie hier beschrieben sowie die Verwendung des Kraftwerkes bzw. des Schwimmkörpers zur Erzeugung brennbarer Kohlenwasserstoffe.

Die in nachfolgend aufgeführten allgemeinen, bevorzugten und besonders bevorzugten Ausführungsformen, Definitionen und Alternativen können beliebig miteinander kombiniert werden und sind Gegenstand der vorliegenden Erfindung. Des Weiteren können einzelne Definitionen / Alternativen entfallen.

Sofern sich aus dem direkten Zusammenhang keine andere Bedeutung ergibt, haben die folgenden Begriffe die hier angegebene Bedeutung:

Der Begriff **Kraftwerk** bezeichnet allgemein einen Verbund verschiedener Anlagen zur Energieerzeugung. Im engeren Sinne werden damit solche Verbundanlagen bezeichnet, die elektrische Energie erzeugen und an ein bestehendes Netz abgeben. Im Rahmen der vorliegenden Erfindung soll der Begriff aber auch solche Anlagenverbunde beschreiben, die einen chemischen Energieträger (insbesondere Methanol, Methan, Dimethylether) zur Verfügung stellen. Ein solches Kraftwerk ist **netzungebunden,** wenn es keine direkte Anbindung an einen Verbraucher aufweist, sondern die erzeugte Energie (bzw. den Energieträger) speichert und so zur Abgabe bereitstellt.

Ein **Schwimmkörper** bezeichnet allgemein jegliche Vorrichtung, welche die nachstehend genannten Anlagenteile aufnehmen kann und sich schwimmend in Regionen konstanter Windverhältnisse / Wellenbewegung aufhalten kann. Insbesondere umfasst dieser Begriff alle Arten von Wasserfahrzeugen die sich aus eigener Kraft bewegen können ("mobile Schwimmkörper", z.B. durch Motorkraft; "Motorschiffe" oder durch Windkraft; "Segelschiffe") oder bewegt werden müssen ("stationäre Schwimmkörper", z.B. "Pontons", "Inseln", "Bojen".). Erfindungsgemäss erfasst der Begriff "durch Windkraft bewegte Schwimmkörper" neben klassischen mit Mast und Segel ausgerüsteten Schiffen auch unkonventionelle Segelschiffe welche beispielsweise einen Segeldrachen oder andere technische Einrichtungen nutzen. Ferner können Schwimmkörper als Einrumpfboot oder Mehrrumpfboote konstruiert sein.

Der Begriff **"Methanol - Synthese"** bzw. "Methanol - Herstellung" bezieht sich auf die Bildung von Methanol aus Wasserstoff und Kohlendioxid bzw. Kohlenmonoxid. Zur Veranschaulichung werden die folgenden Bruttogleichungen angegeben:

CO2 + 3H2 -> H3COH + H2O;

CO + 2H2 -> H3COH

Der benötigte Wasserstoff kann ebenfalls in situ gebildet werden, was zur folgenden Bruttogleichung führt:

CO2 + 2H2O -> H3COH + 3/202.

Der Begriff **"Methan - Synthese"** bzw. "Methan
- Herstellung" bezieht sich auf die Bildung von Methan aus Wasserstoff und Kohlendioxid bzw. Kohlenmonoxid. Zur Veranschaulichung werden die folgenden Gleichungen angegeben:

   4H2O -> 4H2 + 202

   CO2 + 4H2 -> CH4 + 2H2O

   ergibt als Bruttoreaktion:

   CO2 + 2H2O -> CH4 + 202.

Der Begriff **"Dimethylether (DME) - Synthese"** bzw. "DME - Herstellung" bezieht sich auf die Bildung von DME aus Wasserstoff und Kohlendioxid bzw. Kohlenmonoxid. Zur Veranschaulichung werden die folgenden Bruttogleichungen angegeben:

3CO + 3H2 -> H3C-O-CH3 + CO2

2CO2 + 3H2O -> H3C-O-CH3 + 302.

Das gemäss diesen Reaktionen gebildeten chemischen Energieträger (Methanol, DME, Methan) können in verschiedenen Reinheiten vorliegen und müssen nicht vollständig chemisch rein erzeugt werden, d.h. Nebenprodukte, Verunreinigungen aus den vorhergehenden Reaktionsschritten und / oder dem Ausgangsmaterial usw. können enthalten sein. Der erfindungsgemäss zur Verfügung gestellte chemische Energieträger ist geeignet, ggf. nach weiteren Reinigungsschritten, als Energieträger in Verbrennungskraftmaschinen oder Brennstoffzellen oder als Ausgangsmaterial in chemischen Synthesen verwendet zu werden.

Die Erfindung wird weiterhin illustriert durch die Figuren.

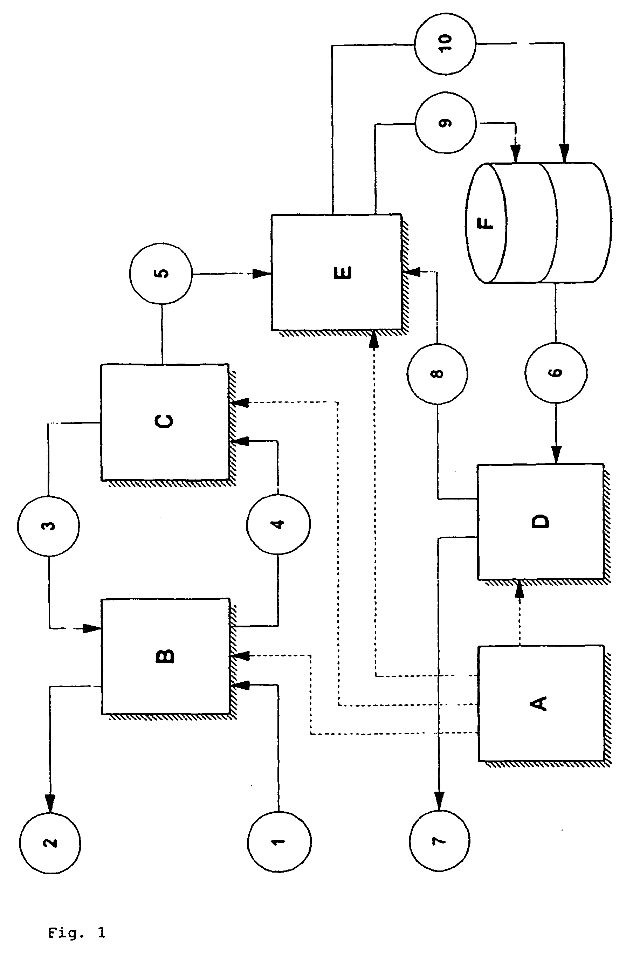

Fig. 1 zeigt eine schematische Darstellung eines erfindungsgemässen Kraftwerkes wobei die Bezugszeichen die folgende Bedeutung haben:

### Aggregate

A Windkraft- oder Photovoltaikanlage
B CO2 Absorptionsanlage
C CO2 Desorptionsanlage
D H2 Syntheseanlage
E katalytische Methanol-Syntheseanlage
F Speicheranlage

### Stoffströme

1 Luft CO2 reich
2 Luft CO2 arm
3 Waschlauge ungesättigt
4 Waschlauge gesättigt
5 CO2
6 H2O
7 02
8 H2
9 Methanol
10 H2O

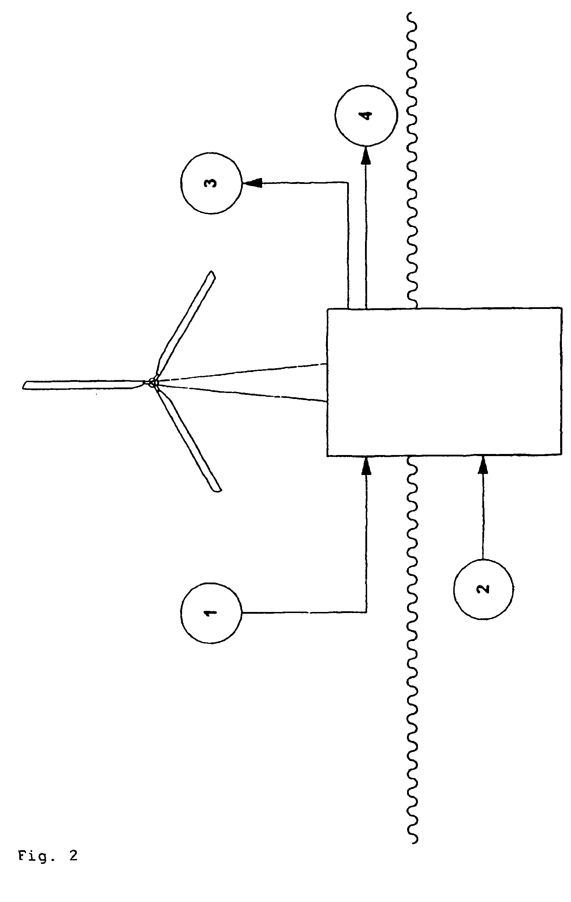

Fig. 2 zeigt eine schematische Darstellung eines erfindungsgemässen Schwimmkörpers enthaltend ein Kraftwerk zur Methanolherstellung wobei die Bezugszeichen die folgende Bedeutung haben:
1 Luft CO2 reich
2 H2O
3 Luft CO2 arm
4 Methanol

Fig. 3 zeigt eine besondere Ausgestaltung der Windkraftanlage, in welcher die Windkraftanlage und die CO2-Absorptionsanlage miteinander kombiniert sind, indem die Rotorblätter der WKA mit mikroporösen Membranen zur CO2-Absorption versehen sind. Hierbei zeigt 1 den CO2-armen Vorlauf und 2 den CO2 reichen Rücklauf der Waschlauge.

In einem **ersten Aspekt** betrifft die Erfindung daher eine netzungebundenes Kraftwerk, dadurch gekennzeichnet, dass es die folgenden, in Ihrer Kapazität aufeinander abgestimmten Anlagen ("Module") enthält: a) Windkraftanlage, Wasserkraftanlage, solarthermische Anlage und / oder Photovoltaikanlage zur Erzeugung elektrischer Energie für den Betrieb der Anlagen b) bis f); b) CO2 - Absorptionsanlage zur Absorption von atmosphärischem CO2; c) CO2 - Desorptionsanlage zur Desorption des in b) gewonnenen CO2; d) elektrochemische oder solarthermische H2 - Syntheseanlage zum Betrieb der Anlage e); e) Syntheseanlage ausgewählt aus der Gruppe katalytische Methanol-Synthese, katalytische DME-Synthese, katalytische Methan-Synthese; f) Speicheranlage ausgewählt aus der Gruppe Methanol - Speicheranlage, DME - Speicheranlage, Methan - Speicheranlage.

Ein derartiges Kraftwerk bietet die Möglichkeit, die in Windkraft, Wasserkraft und/oder Sonnenenergie vorliegende Energie nahezu ortsunabhängig zu ernten und zu speichern. Dadurch eröffnet sich ein großes Potential an möglichen Standorten. Der chemische Energieträger (d.h. das Speichermedium Methanol, DME, Methan) wird dabei aus seinen Verbrennungsprodukten (CO2 und H2O) synthetisiert, bildet also einen geschlossenen Stoffkreislauf.

Methanol ist ein Energieträger mit vergleichsweise hoher Energiedichte, welcher leicht zu handhaben ist und langfristig lagerbar ist. Ferner kann das erfindungsgemäss gebildete Methanol unschwer in die bestehenden Energieversorgungs-Systeme integriert werden, als Chemie-Rohstoff verwendet, oder in Brennstoffzellen (z.B. in DMFC-Zellen) direkt eingesetzt werden. DME und Methan sind ebenfalls Energieträger mit vergleichsweise hoher Energiedichte, welche leicht zu handhaben und lagerbar sind. Ferner können die erfindungsgemäss gebildeten chemischen Energieträger unschwer in die bestehenden Energieversorgungs-Systeme integriert werden, als Chemie-Rohstoff verwendet, oder in Brennstoffzellen direkt eingesetzt werden.

Im Gegensatz zu bekannten Windkraft-, Wasserkraft- oder Photovoltaik-Anlagen, welche in bestehende Netze integriert sind, entstehen auch keine Probleme der Netzwerksteuerung, da keine Spitzenlasten oder Stillstandzeiten auszugleichen sind. Vielmehr ist es möglich, durch Umwandlung des gebildeten chemischen Energieträgers in elektrische Energie einen Netzausgleich zu schaffen.

Die Erfindung betrifft insbesondere die spezielle Abstimmung der Synthesetechnologie auf die Leistung der Windkraft- Wasserkraft- und/oder Photovoltaikanlage; die funktionale Integration von Komponenten der Syntheseanlage in die Windkraft- Wasserkraft- und/oder Photovoltaikanlage; die spezifische Auswahl und Ausgestaltung der einzelnen Anlagen.

Im Folgenden soll die Erfindung weiter erläutert werden und vorteilhafte Ausführungsformen dargestellt werden.

**Allgemein:** Es versteht sich, dass die einzelnen Anlagen/Module a) bis f) in Ihrer Dimensionierung aufeinander abgestimmt sein müssen. Eine derartige Auslegung liegt im Rahmen ingenieurtechnischer Routinetätigkeit. Ferner kann es sich aus Verfahrenstechnischer, Sicherheitstechnischer oder Produktionstechnischer Sicht als vorteilhaft erweisen, die Anlagen a) bis f) mehrfach auszuführen. So kann es z.B. vorteilhaft sein, mehrere Windkraftanlagen, mehrere Syntheseanlagen und mehrere Speicheranlagen vorzusehen. Derartige Variationen liegen im Rahmen der üblichen Ingenieurstätigkeit und sind von der vorliegenden Erfindung mit umfasst. Ferner müssen die einzelnen Elemente der Anlagen ggf. den Bedingungen auf See angepasst sein. Derartige Anpassungen sind bereits aus angrenzenden Gebieten bekannt, z.B. im Zusammenhang mit Ölbohrinseln, auf denen auch eine Weiterverarbeitung des Rohöls stattfindet, oder auf Tankschiffen, welche gefährliche Güter wie Erdgas, H2O2, H2S04 und ähnliches transportieren.

**Kombination von Anlagen / Modulen:** Wie nachstehend noch weiter ausgeführt, können die Anlagen a) bis f) auch als kombinierte Anlagen ausgeführt werden. So kann die Windkraftanlage (a) durch spezielle Ausgestaltung auch die Funktion der CO2-Absorptionsanlage (b) teilweise oder vollständig übernehmen. Ferner können die elektrochemische H2 - Syntheseanlage (d) und die katalytische Methanol - Syntheseanlage (e) kombiniert sein, bspw. in Form einer umgekehrten Direct Methanol Fuel Cell (DMFC). Des Weiteren kann die CO2-Desorptionsanlage (c) und die elektrochemische H2 - Syntheseanlage (d) miteinander kombiniert sein. Auch können die CO2-Absorptions- und Desorptionsanlage kombiniert oder funktionell gekoppelt sein. Derartige kombinierte bzw. zusammengefasste Anlagen sind von der vorliegenden Erfindung mit umfasst.

Ferner kann es für die optimale Stoff- und Energiekopplung der Anlage sinnvoll sein, bestimmte Ausführungsformen der einzelnen Anlagen a) bis f) miteinander zu kombinieren. So ist es bspw. vorteilhaft, eine thermische CO2- Desorptionsanlage mit einer solarthermischen H2-Syntheseanlage zu kombinieren. Weiterhin ist es bspw. vorteilhaft, eine CO2-Desorptionsanlage welche nach dem Prinzip reduktiven alkalischen Niederdruck-Elektrolyse arbeitet mit einer Methanol-Syntheseanlage auf Basis von Synthesegas zu kombinieren. Sodann ist es bspw. vorteilhaft, die CO2-Desorptionsanlage, welche nach dem Prinzip der Chlorierung arbeitet, mit einer H2-Syntheseanlage zu kombinieren, welche nach dem Prinzip der Chlor-Alkali-Elektrolyse arbeitet. Im Prinzip können die nachstehenden Module beliebig miteinander kombiniert werden. Es ergeben sich, z.B. aus wirtschaftlichen oder technischen Überlegungen, besonders bevorzugte Kombinationen, welche Gegenstand der vorliegenden Erfindung sind. Diese Kombinationen sollen anhand des morphologischen Kastens am Ende dieser Beschreibung im Überblick dargestellt werden. Die genannten einzelnen Anlagen werden nachstehend im Detail erläutert.

Schwimmkörper: Der Begriff wurde bereits eingangs erläutert. Schwimmkörper können eigens für den erfindungsgemässen Zweck erstellt werden. Alternativ können bestehende Vorrichtungen umgenutzt werden, z.B. Indem Frachtschiffe oder Bohrinseln mit den entsprechenden Anlagen ausgerüstet werden. Geeignete Schwimmkörper sind z.B. Frachtschiffe und Bojen.

**Anlage zur Erzeugung von Energie (Modul a):** für den hier beschriebenen Prozess ist je nach Ausgestaltung, elektrische und / oder thermische Energie nötig. Diese Energie wird über eine Primäranlage zur Verfügung gestellt. Prinzipiell sind alle bekannten Anlagen geeignet, die elektrische bzw. thermische Energie zur Verfügung stellen. Als vorteilhafte Anlagen sind Windkraftanlagen, Wasserkraftanlagen, Photovoltaikanlagen und solarthermische Anlagen zu nennen, wobei Windkraftanlagen und solarthermische Anlagen besonders hervorzuheben sind. Ferner sind Wasserkraftanlagen besonders hervorzuheben.

Unter Windkraftanlagen ("WKA"), (Modul a1) sind im Zusammenhang mit der vorliegenden Erfindung alle Anlagen zu verstehen, die Windenergie in elektrische Energie umwandeln können. Typischerweise enthalten WKAs einen Rotor aus Nabe und Rotorblättern, welcher auf einem Mast montiert ist und mit einem Generator gekoppelt ist; der Begriff umfasst somit WKAs mit vertikal oder horizontal drehenden Rotorblättern. Ferner können WKAs mit oder ohne Getriebeunterstützung eingesetzt werden.

Unter Wasserkraftanlage (Modul a2) sind im Zusammenhang mit der vorliegenden Erfindung alle Anlagen zu verstehen, welche entweder die kinetische Energie des Wassers nutzbar machen oder die relative Bewegung von Schwimmkörper zum Wasser nutzbar machen. Bevorzugt wäre daher auch ein bewegter Schwimmkörper ("Segelschiff") welcher durch im wesentlichen ruhendes Wasser bewegt wird. Bevorzugt ist ferner ein Schwimmkörper, welcher die Energie der Wasserwellen in elektrische Energie umsetzen kann.

Unter Photovoltaikanlagen (Modula a3) sind im Zusammenhang mit der vorliegenden Erfindung alle Anlagen zu verstehen, die in der Sonnenstrahlung enthaltene Sonnenenergie auf direktem Weg in elektrische Energie umwandeln können.

Unter Solarthermischer Anlage (Modul a4) sind im Zusammenhang mit der vorliegenden Erfindung alle Anlagen zu verstehen, welche die Strahlungsenergie der Sonne thermisch nutzbar machen.

Die vorstehend genannten Anlagen (Module) sind an sich bekannt und kommerziell erhältlich bzw. nach bekannten Methoden auszulegen und herzustellen.

In einer vorteilhaften Ausführungsform wird eine vertikal drehende WKA ohne Getriebeunterstützung verwendet. Diese Ausführungsform hat den Vorteil, dass eine besonders wartungsarme WKA und problemlos montierbare WKA verwendet wird.

In einer alternativen Ausführungsform wird eine horizontal drehende WKA mit Getriebeunterstützung verwendet. Diese Ausführungsform hat den Vorteil, dass eine besonders energieeffiziente und flexible WKA verwendet wird.

In einer weiteren alternativen Ausführungsform wird eine solarthermische Anlage und ggf. eine WKA verwendet. Diese Ausführungsform ist von Vorteil, wenn die nachfolgenden Anlagen einen geringen Bedarf an elektrischer Energie und einen hohen Bedarf an thermischer Energie zeigen.

In einer weiteren alternativen Ausführungsform der Erfindung wird eine Wellenkraftmaschine (z. B. vom Typ eines "Pelamis" oder "OPT Powerbuoy") verwendet, welche die Auf- und Ab- Bewegung der Wellen nutzt, um die daraus resultierende Energie in elektrische Energie zu verwandeln. Solche Wellenkraftmaschinen werden entweder verankert oder mit anderen Mitteln, bspw. via Segel, positioniert. Ein Segelboot mit daran befestigten OFT Powerbuoys ist somit eine spezifische Ausführungsform von o.g. Wasserkraftanlage.

In einer weiteren alternativen Ausführungsform der Erfindung wird eine im Wasser angeordnete Turbine verwendet (z.B. wie sie in Gezeitenkraftwerken verwendet werden) welche eine Strömung im Wasser, bspw. eine Meeresströmung, nutzt. Solche Turbinen können bspw. an ortsfesten Schwimmkörpern befestigt werden. Die Erfindung betrifft daher auch ein modulares Kraftwerk, bei dem der Schwimmkörper ortsfest ist und der eine im Wasser angeordnete Turbine aufweist.

In einer weiteren alternativen Ausführungsform wird eine im Wasser angeordnete Turbine verwendet, welche am Rumpf eines Segelboots befestigt ist. In dieser Ausführungsform wird besagtes Segelboot durch Windkraft relativ zum Wasser bewegt und treibt so die Turbine zur Stromerzeugung an. Besagtes Seegelboot kann dabei entweder ein klassisches Segelboot mit einem oder mehreren Rümpfen sein, oder ein Boot welches mit nichtkonvnetionellen Einrichtungen zum Windvortrieb, bspw. einem Drachen, ausgerüstet ist. Die Erfindung betrifft daher auch ein modulares Kraftwerk, bei dem der Schwimmkörper ein Segelboot ist welches eine oder mehrere im Wasser angeordnete Turbinen aufweist.

**CO2 - Absorptionsanlage (Modul b):** Zahlreiche Anlagen und Prozesse zur Absorption von atmosphärischem CO2 sind bekannt. Erfindunggemäss sind CO2 - Absorptionsanlagen Anlagen auf Basis einer Waschlauge (Nasschemische Anlagen, Modul b1), oder ohne die Verwendung von Waschlaugen ("Trockenchemische Anlagen", Modul b2) umfasst.

**Modul b1:** Im Rahmen der vorliegenden Erfindung sind solche Anlagen bevorzugt, bei denen Luft (welche ca. 350 ppm CO2 enthält) mit einer Waschlauge, insbesondere eine alkalischen wässrigen Lösung, bevorzugt ein Alkalihydroxid wie NaOH oder KOH, in Kontakt gebracht wird, wobei sich Carbonat und/oder Hydrogencarbonat bildet, vgl Weimer et al, Energy Convers. Mgmt. 1996, p.1351 ff, welches durch Referenz in diese Beschreibung aufgenommen wird (insbesondere: Kapitel 3.3.2). Zur Erzeugung von 11 Methanol wird entsprechend das CO2 von 1700m3 Luft benötigt. Um eine Syntheseanlage mit einer Primärleistung von 600kW mit CO2 zu versorgen, muss pro Stunde 120000m3 Luft der Absorptionsanlage zugeführt werden. Geeignete Waschlaugen reagieren reversibel mit CO2 und sind allgemein bekannt, beispielhaft sei die bereits erwähnte NaOH-Lösung genannt. Die Reaktion kann z.B. gemäss folgender Gleichungen im wässrigen Medium erfolgen:

2NaOH + CO2 -> Na2CO3 + H2O

NaOH + CO2 -> NaHCO3,

wobei Carbonat und Hydrogencarbonat miteinander im Gleichgewicht stehen. Sofern im Zusammenhang mit der vorliegenden Erfindung auf Carbonat als Reaktionspartner abgestellt wird, ist die entsprechende Reaktion des Hydrogencarbonates mit umfasst, und lediglich aus Gründen der Übersichtlichkeit nicht mit aufgeführt.

In einer Ausführungsform kann die Absorption im offenen Kontakt in einem mit Füllkörpern versehenen Waschturm erfolgen. Dabei wird der Luftstrom ggf. zunächst befeuchtet und dann im Gegenstrom zur alkalischen Waschlauge durch diesen mit Füllkörpern versehenen Absorptionsturm geleitet.

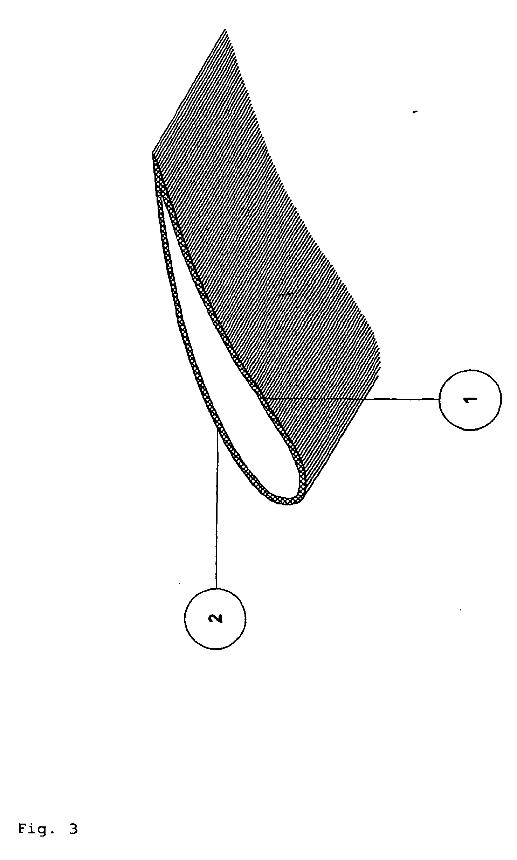

In einer weiteren vorteilhaften Ausführungsform ist die Absorptionsanlage vom Typ eines Venturi-Turms. Solche Venturi-Türme enthalten Mittel zur Zuführung der wässrigen alkalischen Lösung, Mittel zur Tropfenbildung / Vernebelung dieser Lösung im oberen Bereich des Turms, Mittel zur Abführung der gebildeten Carbonat / Hydrogencarbonat - Lösung im unteren Bereich des Turms. Geeignete Venturi - Türme sind aus US 4339547 bekannt, welche durch Referenz in diese Beschreibung aufgenommen wird (insbesondere: Fig. 3a, b; Spalte 2, Z. 63 - Sp.3, Z. 38)

In einer weiteren vorteilhaften Ausführungsform enthält die Absorptionsanlage mikroporöse Holfasermembranen, insbesondere hydrophobe mikroporöse Holfasermembranen, welche alkalischen Absorptionsflüssigkeit enthalten und mit einem Luftstrom in Kontakt gebracht werden um so atmosphärisches CO2 zu absorbieren. Solche Membranen sind bekannt und bspw. von Hoechst - Celanese kommerziell erhältlich. Die Konstruktion und Dimensionierung entsprechender Absorber ist an sich bekannt und bspw. in Stucki et al, Int. J. Hydrogen Energy, 1995, S.653 ff beschrieben. Dieses Dokument, (insbesondere S. 656, "experimental") werden durch Referenz in diese Beschreibung aufgenommen. In einer vorteilhaften Ausführungsform werden diese mikroporösen Holfasermembranen mit einem hydgroskopischen Material, bspw. MgS04, belegt. Diese Massnahme reduziert die Verdunstung des zirkulierenden Wassers der Waschlösung.

In einer besonderen Ausführungsform werden die Windkraftanlage und die CO2-Absorptionsanlage miteinander kombiniert, indem die Rotorblätter und / oder der Mast der WKA mit den vorstehend genannten mikroporösen Membranen versehen sind. Diese Membranen werden derartig auf Mast und/oder Rotorblatt montiert, dass sie einerseits mit der Luft im direkten Kontakt sind und andererseits die Waschlauge von und zur CO2-Desorptionsanlage zirkulieren kann. Die Erfindung betrifft daher auch Windkraftanlagen und netzungebundene Kraftwerke wie hier beschrieben, bei denen die Rotorblätter und / oder der Mast mit hydrophoben mikroporösen Membranen zur CO2 Absorption versehen sind.

In einer weiteren vorteilhaften ausgestaltung der hier beschriebenen Nasschemischen Absorptionsanlagen wird der Waschlauge eine die Verdunstung reduzierende Komponente zugegeben. Geeignete Komponenten haben eine Dampfdruck - reduzierende Wirkung. Solche Komponenten sind an sich bekannt, als spezifisches Beispiel sei Magnesiumsulfat genannt. Die effektiv notwendige Konzentration solcher Komponenten hängt von der Konstruktion der Anlage, den Verwendeten Einsatzstoffen und den Betriebsbedingungen ab. Die Bestimmung geeigneter Betriebsparameter kann anhand entsprechender Versuche bestimmt werden, als Anhaltspunkt sind 0.1 - 10 Gew-% MgS04 in der Waschlauge geeignet.

**Modul b2:** Im Rahmen der vorliegenden Erfindung sind ferner solche Anlagen bevorzugt, bei denen Luft mit festem CaO bei vergleichsweise niedrigen Temperaturen (insbesondere <100°C) kontaktiert wird, wobei sich CaCO3 bildet. Das genannte CaO liegt dabei vorteilhaft in feinverteilter Form, bspw. als Nanopartikel vor. Entsprechende Apparate und Anlagen, welche mittels eines festen Absorbens Gase absorbieren sind an sich bekannt und können nach bekannten Methoden ausgelegt werden. Diese Trockenchemische Anlage wird bevorzugt mit der nachstehend genannten Trockenchemischen CO2 - Desorptionsanlage kombiniert.

**CO2 - Desorptionsanlage (Modul c):** Anlagen zur Freisetzung von CO2 aus Waschlaugen, insbesondere aus carbonathaltigen wässrigen Waschlaugen, sind allgemein bekannt (Modul c1). Anlagen zur Freisetzung von CO2 aus Feststoffen, insbesondere aus carbonathaltigen Feststoffen, sind ebenfalls allgemein bekannt (Modul c2). Eine solche Freisetzung kann auf physiko-chemischen, elektrochemischen oder chemischen Weg erfolgen und steht in engem Zusammenhang mit der durch die CO2-Absorption gebildeten Verbindungen.

In einer Ausführungsform erfolgt die CO2-Freisetzung auf elektrochemischen Weg durch Elektrolyse der gebildeten Carbonatlösung in einer elektrochemischen Membranzelle unter Bildung von CO2 und H2 ("alkalische Niederdruck-Elektrolyse", Modul c11). Solche Zellen sind an sich bekannt; im Wesentlichen kann die Reaktion durch folgende Gleichung beschrieben werden:

Na2C03 + 4H2O -> 3H2 + 3/202 + CO2 + 2NaOH

Der Vorteil dieser Ausführungsform liegt darin, dass die CO2 - Desorptionsanlage und die H2 - Syntheseanlage miteinander kombiniert sind.

In einer alternativen Ausführungsform erfolgt die CO2-Freisetzung auf elektrochemischen Weg durch Elektrolyse der gebildeten Carbonatlösung in einer elektrochemischen Membranzelle unter Bildung von CO (bspw. unter Verwendung einer Solid Oxide Electrolysis Cell, SOEC, Modul c12). Eine derartige Anlage wird im Zusammenhang mit der vorliegenden Erfindung ebenfalls als CO2-Desorptionsanlage betrachtet, da das aus der Luft gebundene CO2 wieder abgegeben wird, im speziellen fall in der Form von CO + 1/2O2. Solche Zellen sind an sich bekannt; im Wesentlichen kann die Reaktion durch folgende Gleichung beschrieben werden:

Na2C03 + H2O -> 1/202 + CO + 2NaOH

Der Vorteil dieser Ausführungsform liegt darin, dass CO gebildet wird, welches in der Nachfolgenden Methanolsyntheseanlage zu Methanol oder in einer DME Syntheseanlage zu DME umgesetzt wird, ohne Wasser als Nebenprodukt zu bilden. Ferner ist bei dieser Umsetzung der Strombedarf vergleichsweise niedriger.

In einer alternativen Ausführungsform erfolgt die CO2-Freisetzung auf chemischen Weg durch Säuerung der Waschlauge aus Anlage b) mit ggf. anschliessender Elektrodialyse (Modul c13). Die Desorption durch Zugabe einer Säure, bspw. wässrige Schwefelsäure, ist allgemein bekannt und kann in gängigen Apparaten, bspw. einem Rührreaktor erfolgen. Das in dieser Reaktion gebildete Salz kann in einer nachfolgenden Elektrodialyse wieder aufgearbeitet werden. Im Wesentlichen können die Reaktion durch die Folgenden Gleichungen beschrieben werden:

Na2C03 + H2SO4 -> Na2S04 + CO2 + H2O

Na2SO4 + H2O -> NaOH + H2SO4.

Der Vorteil dieser Ausführungsform liegt in der einfachen und sicheren CO2-Desorption.

In einer alternativen Ausführungsform erfolgt die CO2-Freisetzung auf physiko-chemischen (thermischen) Weg durch Calcinierung (Modul c2). Dabei wird ein (Erd-)Alkalicarbonat, bevorzug Calciumcarbonat, so weit erhitzt, dass CO2 abgespalten wird. Geeignete Reaktoren für diese Reaktion sind bekannt, bspw. Drehrohröfen oder Fliesbettreaktoren. Die benötigten (Erd-)alkalicarbonate können direkt aus der CO2-Absorptionsanlage (auf Nasschemischen oder Trockenchemischen Weg) erhalten werden oder durch Umsetzung mit Na2CO3 (aus der Absorptionsanlage) erzeugt werden. Die bei der Calcinierung entstehenden Metalloxide können durch Reaktion mit Wasser recycliert werden (für Nasschemische Verfahren) oder ohne weitere chemische Umsetzung (für Trockenchemische Verfahren) recycliert werden. Im Wesentlichen können die Reaktion des Nasschemischen Verfahrens durch folgende Gleichungen, welche die Bereitstellung des Carbonates, die Calcinierung und die Recyclierung zusammenfassen, beschrieben werden:

Na2CO3 + Ca(OH)2 -> CaC03 + 2NaOH

CaCO3 -> CaO + CO2

CaO + H2O -> Ca(OH)2

Ein Vorteil dieser Ausführungsform ist, dass ein besonders feinteiliges Carbonat für die weitere Reaktionssequenz zur Verfügung gestellt wird. Ein weiterer Vorteil dieser Ausführungsform ist es, das auf elektrische Energie zur Durchführung der Reaktion verzichtet werden kann.

Im Wesentlichen können die Reaktion des Trockenchemischen Verfahrens durch folgende Gleichungen, welche die Bereitstellung des Carbonates, die Recyclierung zusammenfassen, beschrieben werden:

CaO (s) + CO2 (g) -> CaC03 (s)

CaCO3(s) -> CaO (s)+ CO2 (g)

Dabei liegen die Feststoffe in feinteiliger Form, bevorzugt als Nanopartikel, vor.

In einer alternativen Ausführungsform des Moduls c2 erfolgt die CO2-Freisetzung aus CaC03 solarthermisch bei 500 - 2000°C, bevorzugt ca. 1000°C. Solarthermie-Anlagen sind bekannt; geeignet sind im Prinzip alle derartigen Anlagen, die durch Bündelung oder Fokussierung die gewünschten Temperaturen erzeugen; bspw. mittels Parabolspiegel oder Fresnel-Linsen.

In einer weiteren Ausgestaltung des Moduls c2 kann im Reaktionsraum der CO2-Partialdruck abgesenkt werden, um so die Freisetzung günstig zu beeinflussen. Eine Herabsetzung des CO2-Partialdruckes ist bspw. möglich, indem H2 in den Reaktionsraum eingeleitet wird.

In einer alternativen Ausführungsform erfolgt die CO2-Freisetzung auf chemischen Weg durch Umsetzung der Carbonat-Lösung mit Chlor ("Desorption durch Chlorierung", Modul c14), ggf. in Gegenwart eines Katalysators, unter Bildung einer Chlorid/Hypochlorid - Lösung und CO2. Diese Reaktion und entsprechende Apparate ("Stripping - Türme") sind an sich bekannt. Das benötigte Chlor wird elektrochemisch recycliert; bspw. indem die gebildete Chlorid/Hypochlorid-Lösung zunächst reduziert wird und anschliessend der H2-Syntheseanlage zugeführt wird in der eine Chlor-Alkali-Elektrolyse durchgeführt wird. Im Wesentlichen können die Reaktion durch folgenden Gleichungen, welche die Desorption, die Reduktion sowie H2-Synthese/C12 recycling zusammenfassen, beschrieben werden:

Na2CO3 + C12 -> NaCl + NaOCl + CO2

NaOCl + NaCl -> 2NaCl + 1/2 02

2NaCl + 2H2O -> 2NaOH + Cl2 + H2

Eine derartige Ausführungsform ist bspw. dann angezeigt, wenn die H2 - Syntheseanlage so ausgestaltet ist, dass Chlor als Nebenprodukt erzeugt wird.

In einer alternativen Ausführungsform kann die CO2-Freisetzung auf physiko-chemischen Weg in einer Anlage durch Druckverminderung ("Entgasungsanlage") erfolgen. Solche Entgasungsanlagen sind an sich bekannt. Der Vorteil dieser Ausführungsform ist, dass eine technisch einfache und robuste Anlage bereitgestellt wird. Diese Ausführungsform ist vor allem dann vorteilhaft, wenn in der CO2-Absorptionsanlage eine Waschlauge eingesetzt wurde, welche mit dem CO2 eine vorwiegend physikalische Bindung oder schwache chemische Bindung eingeht, d.h. im wesentlichen absorptive oder koordinative Effekte auftreten.

**H2 - Syntheseanlage** (Modul d): Die Bereitstellung des benötigten Wasserstoffs kann auf elektrochemischen (Modul d1) und/oder solarthermischem (Modul d2) Weg erfolgen, wobei elektrochemische H2 - Syntheseanlagen bevorzugt sind. Prinzipiell sind alle bekannten elektrochemischen Zellen geeignet, welche wässrige Lösungen unter Bildung von Wasserstoff elektrolysieren. Solche Zellen sind bekannt, weitgehend untersucht und können vom Fachmann ausgelegt und in den Gesamtprozess integriert werden. Eine Anpassung ist bspw. auf die vorhergehende CO2-Desorptionsanlage sinnvoll bzw. notwendig. Des weiteren sind prinzipiell alle bekannten solarthermischen Prozesse geeignet, welche zur H2 - Freisetzung führen. Auch hier ist eine Anpassung an die weiteren Anlagen sinnvoll bzw. notwendig. Ferner sind bei allen H2 - Syntheseanlagen die Betriebssicherheit, Investitionskosten und Effizienz wichtige Parameter.

**Modul d1:** In einer vorteilhaften Ausführungsform erfolgt die H2 - Herstellung mittels PEM Elektrolyse (Proton Exchange Membrane). Diese Technologie ist Stand der Technik und lässt sich leicht im geforderten Massstab realisieren.

In einer weiteren vorteilhaften Ausführungsform erfolgt die H2 - Herstellung mittels Chlor-Alkali-Elektrolyse einer wässrigen Kochsalzlösung. Im Prinzip sind alle dafür bekannten Elektrolysezellen geeignet. Diese Ausführungsform ist besonders vorteilhaft, wenn die CO2-Desorption durch Chlorierung erfolgt.

In einer weiteren vorteilhaften Ausführungsform erfolgt die H2 - Herstellung mittels Hochtemperatur-Elektrolyse von Wasser unter Bildung von Wasserstoff und Sauerstoff. Im Prinzip sind alle dafür bekannten Elektrolysezellen geeignet. Vorteilhaft wird das eingesetzte Wasser zunächst entsalzt, bspw. mittels Ionenaustauscher oder Destillation. Diese Ausführungsform ist besonders vorteilhaft, wenn die CO2-Desorption nicht durch Chlorierung erfolgt.

In einer weiteren vorteilhaften Ausführungsform wird die bei der CO2 Absorption gebildete wässrige Carbonat / Hydrogencarbonat-Lösung direkt einer Elektrolyse zugeführt, wobei CO2 und 02 an der Anode und H2 an der Kathode gebildet wird und eine Hydroxid-haltige Lösung entsteht, welche für die CO2 Absorption recycliert werden kann ("alkalische Niederdruck-Elektrolyse"). Solche Elektrolyse-Zellen sind an sich bekannt und in US3135673 beschrieben, dessen Inhalt durch Bezugnahme in diese Beschreibung aufgenommen wird. Im Wesentlichen können die Reaktion durch folgende Gleichung beschrieben werden:

Na2C03 + 4H20 -> 3H2 + 3/202 + CO2 + 2NaOH.

Vorteilhaft kann diese Elektrolysezelle so konstruiert werden, dass in einer Dreiteiligen, durch Diaphragmen getrennten Zelle H2 und O2 an den Kathoden- bzw. Anodenräumen entnommen werden können und das gebildete CO2 im dazwischen liegenden Bereich entnommen werden kann.

**Modul d2:** In einer weiteren vorteilhaften Ausführungsform wird der benötigte Wasserstoff durch solarthermische Prozesse ohne Elektrizität hergestellt. Dies kann z.B. durch thermische Dissoziation von ZnO bei Temperaturen über 2000 K erfolgen wobei Zn und ½ 02 gebildet werden. Zn reagiert mit H2O unter der Bildung von ZnO wobei H2 freigesetzt wird. Diese Ausführungsform ist dann vorteilhaft, wenn Sonnenstrahlung in hoher Intensität zur Verfügung steht. Eine solche solarthermische H2 - Syntheseanlage kann vorteilhaft mit einer CO2 - Desorptionsanlage kombiniert werden, welche das CO2 aufgrund thermischer Prozesse freisetzt, wie vorstehend beschrieben. In dieser Ausführungsvariante kann die für alle endothermen Prozesse benötigte Reaktionsenergie thermisch zur Verfügung gestellt werden. Dabei werden für die CO2-Desorption und die H2-Synthese thermische Energie bereitgestellt; die CO2 - Absorption und die Synthese von Methanol, DME, Methan sind exotherme Reaktionen. Der besondere Vorteil dieser Ausführungsform wäre daher im geringen Bedarf an elektrischer Energie und dem hohen Gesamtwirkungsgrad des Kraftwerkes zu sehen. Entsprechend wäre ein solches Kraftwerk in Regionen mit hoher Sonneneinstrahlung, bspw. in Wüstenregionen, vorteilhaft zu installieren.

**Syntheseanlage zur Erzeugung brennbarer Kohlenwasserstoffe (Modul e).** Die Synthese von brennbaren Kohlenwasserstoffen, insbesondere Methanol, Methan, DME, sowie entsprechende Anlagen sind im Stand der Technik bekannt. Vorteilhaft werden Anlagen verwendet, die auf katalytischen Syntheseprozessen beruhen.

**Methanol - Syntheseanlage** (Modul e1): Wie bereits ausgeführt werden von der vorliegenden Erfindung Methanol - Syntheseanlagen umfasst, die entweder i) Kohlendioxid (CO2) und Wasserstoff oder ii) Kohlenmonoxid (CO)und Wasserstoff oder iii) Kohlendioxid und Wasser in Methanol überführen. Diese Anlagen können so ausgelegt werden, dass bei einfachem Durchsatz praktisch vollständige Umsetzung zu Methanol erfolgt. Alternativ können diese Anlagen auch so ausgelegt oder betrieben werden, dass nur eine teilweise Umsetzung des eingesetzten CO bzw. CO2 erfolgt. In diesem Fall wird nicht umgesetztes Ausgangsmaterial entweder nach Abtrennung des Produktes (Methanol) recycliert. oder (insbesondere im Fall von CO2) in die Umgebung abgegeben. Bevorzugt erfolgt eine vollständige (bzw. praktisch vollständige) Recyclierung ohne Abgabe an die Umgebung.

In einer vorteilhaften Ausführungsform erfolgt die Methanol - Synthese aus den Komponenten CO2 und H2. Anlagen zur Umsetzung von CO2 und H2 zu Methanol sind allgemein bekannt. Typischerweise sind diese Anlagen so ausgelegt, dass im Reaktor die Umsetzung bei 50 - 100 bar, 200 - 300 °C erfolgt. Die Reaktoren können als Festbett oder Wirbelschichtreaktoren ausgelegt werden. Geeignete Katalysatoren sind bspw. Cu - dotierte Feststoffkatalysatoren. Die Methanolsynthese kann einstufig oder mehrstufig durchgeführt werden. Diese Umsetzung kann durch folgende Reaktionsgleichung veranschaulicht werden:

CO2 + 3H2 -> H3COH + H2O

Diese Variante ist besonders vorteilhaft, da derartige kommerziell erhältliche Anlagen in ihrer Dimensionierung auf die durch typische Windkraftanlagen bereitgestellte elektrische Energie abgestimmt werden können. Vorteilhaft ist in dieser Anlage eine Destillationseinheit vorgesehen, welche die teilweise oder vollständige Abtrennung des gebildeten Wassers vom Methanol ermöglicht. Die Erfindung betrifft somit auch ein Kraftwerk wie hier beschrieben, bei dem die Methanolsyntheseanlage einen Cu-haltigen Katalysator (zur Umsetzung von CO2 und H2) enthält, und der ggf. eine Destillationseinheit (zur teilweisen oder vollständigen Abtrennung des erzeugten Methanols und zur Rückführung des Wassers zur Elektrolyse) zugeordnet ist.

In einer weiteren vorteilhaften Ausführungsvariante erfolgt die Methanol-Synthese aus Synthesegas (ein gasförmiges Gemisch, im wesentlichen enthaltend CO / H2, bevorzugt im molaren Verhältnis 1:2) Anlagen zur Umsetzung von CO und H2 zu Methanol sind allgemein bekannt. Diese Umsetzung kann durch folgende Reaktionsgleichung veranschaulicht werden:

CO + 2H2 -> H3COH

Solche Anlagen werden im Zusammenhang mit der vorliegenden Erfindung als "Methanol/Synthesegas-Anlage" bezeichnet. Bei dieser Variante ist es vorteilhaft, dass kein Wasser als Nebenprodukt gebildet wird, so dass auf eine destillative Trennung von Methanol/Wasser verzichtet werden kann. Für diese Ausführungsform ist es jedoch notwendig, das CO2 der Luft vorgängig zu CO zu reduzieren. Entsprechende Prozesse und Anlagen sind bekannt und können in das hier beschriebene Kraftwerk integriert werden.

In einer Variante enthält die Methanol/Synthesegas-Anlage eine Einheit zur Hochtemperatur-Elektrolyse von CO2. Vorteilhaft enthalten solche Einheiten einen sauerstoffionen leitenden Festelektrolyten, bspw. ZrO/Y2O3. Typische Reaktionstemperaturen sind dabei 800 - 1000°C. Der Einheit zugeführte CO2/H2O - Gemische werden bei Anlegen einer Spannung gemäss nachstehenden Gleichungen reduziert:

CO2 -> CO + 1/202

H2O -> 2H2 + 02.

Die Stöchiometrie des erzeugten Synthesegases ist von der angelegten Spannung, der Kontaktzeit, der Verweildauer, der Temperatur abhängig und kann in einfachen Reihenversuchen optimiert werden. Sofern eine derartige Hochtemperatur - Elektrolyse mit der Methanol/Synthesegasanlage kombiniert wird, kann, je nach Betriebspunkt der Hochtemperatur - Elektrolyseeinheit auf eine separate H2-Syntheseanlage verzichtet werden.

In einer weiteren Variante wird die Methanol / Synthesegas - Anlage mit einer Anlage zur reduktiven alkalischen Niederdruckelektrolyse kombiniert. Diese Anlage stellt das benötigte CO zur Verfügung.

In einer weiteren bevorzugten Ausführungsform erfolgt die Methanol-Synthese gemäss:

CO2 + 2H2O -> H3COH + 3/202.

Diese Anlage wird als "Direkt-Methanol-Anlage" bezeichnet. Dieses Verfahren ist besonders vorteilhaft, da die Komponenten CO2 und H2O direkt verwendet werden können. Bei dieser Variante sind demgemäss Methanol- und Wasserstoff-Syntheseanlage (Anlagen d) und e)) in einer einzigen Anlage kombiniert. Solche Anlagen sind an sich bekannt und z.B. in US 5928806, deren Inhalt durch Bezug in diese Beschreibung aufgenommen wird.

**Methan - Syntheseanlage** (Modul e2): In einer Ausführungsform erfolgt die Methan - Synthese aus den Komponenten CO2 und H2. Anlagen zur Umsetzung von CO2 und H2 zu Methan sind allgemein bekannt. Typischerweise sind diese Anlagen so ausgelegt, dass im Reaktor die Umsetzung bei 1 - 30 bar, bevorzugt bei Normaldruck, 300 - 400 °C erfolgt. Die Reaktoren können als Festbett oder Wirbelschichtreaktoren ausgelegt werden. Geeignete Katalysatoren sind bspw. Ni - dotierte Feststoffkatalysatoren. Die Methanolsynthese kann einstufig oder mehrstufig durchgeführt werden, bevorzugt einstufig. Diese Umsetzung kann durch folgende Reaktionsgleichung veranschaulicht werden:

CO2 + 4H2 -> CH4 + 2H2O

Diese Variante ist besonders vorteilhaft, da sie leicht miniaturisert werden können, dass drucklose Verfahrensvarianten bereits bekannt sind und dass auf eine destillative Aufreinigung verzichtet werden kann. Diese Vorteile können den Nachteil der Speicherung unter Druck teilweise oder vollständige aufheben.

Die Erfindung betrifft somit auch ein Kraftwerk wie hier beschrieben, bei dem die Methansyntheseanlage einen Ni-haltigen Katalysator (zur Umsetzung von CO2 und H2) enthält, und der keine Destillationseinheit zur Produkttrennung zugeordnet ist.

**Dimethylether - Syntheneanlage** (Modul e3): In einer Ausführungsform erfolgt die DME - Synthese aus Methanol unter Abspaltung von Wasser. Anlagen für diese Umsetzung sind allgemein bekannt. Typischerweise sind diese Anlagen so ausgelegt, dass im Reaktor die Umsetzung bei 30 - 80 bar, bevorzugt bei 50 bar, 200 - 300 °C erfolgt. Die Reaktoren können als Festbett oder Wirbelschichtreaktoren ausgelegt werden. Geeignete Katalysatoren sind bspw. Cu / Fe - dotierte Feststoffkatalysatoren. Diese Variante ist besonders vorteilhaft, da mit DME ein Energieträger erzeugt wird, der unter geringem Druck verflüssigt werden kann.

Alternativ kann die DME Synthese auf direktem Weg erfolgen; entsprechende Anlagen und Katalysatoren sind bekannt. Die Bruttogliechungen solcher Reaktionen sind nachforlgend angegeben:

3CO + 3H2 -> H3C-O-CH3 + CO2

2CO2 + 3H2O -> H3C-O-CH3 + 302.

**Speicheranlage (Modul f):** Speicheranlagen für flüssige oder gasförmige brennbare Kohlenwasserstoffe sind an sich bekannt.

**Methanol - Speicheranlage** (Modul f1): Speicheranlagen ("Tanks") geeignet für Methanol sind an sich bekannt. Aufgrund seiner physiko-chemischen Eigenschaften, insbesondere Flammpunkt, Dampfdruck und Lösungseigenschaften müssen Tanks aus geeigneten Materialien bestehen und entsprechende Sicherheitseinrichtungen aufweisen. Solche Materialien und Sicherheitseinrichtungen sind dem Fachmann bekannt. Ferner kann vorgesehen werden, dass die Methanol-Speicheranlage räumlich von den anderen Anlagenteilen getrennt ist. Die Erfindung betrifft daher auch ein Schwimmkörper wie hier beschrieben, bestehend aus zwei einzelnen, miteinander verbundenen Vorrichtungen bei der die eine Vorrichtung nur Anlage f) enthält und die zweite Vorrichtung die anlagen a) - e) enthält.

**Methan - Speicheranlage** (Modul f2): Anlagen zur Speicherung von Methan werden bevorzugt so ausgelegt, dass entweder gasförmiges Methan bei 200bar ("CNG") bei Umbebungstemeperatur oder gasförmiges Methan drucklos und bei Umbebungstemeperatur oder flüssiges Methan und drucklos bei -163°C ("LNG") gespeichert wird. Entsprechende Anlagen sind bereits grosstechnisch im Einsatz. Im Übrigen gelten die für Methanol gemachten Aussagen entsprechend.

**DME - Speicheranlage** (Modul f3): Die für Methanol gemachten Aussagen treffen entsprechend auch für DME zu. DME kann ähnlich wie LPG gespeichert werden, so dass die entsprechenden Anlagen gemäss dieser Erfindung eingesetzt werden können. Bevorzugt sind DME - Speicheranlagen so ausgelegt, das flüssiges DME bei 5 bar und Umgebungstemperatur gespeichert wird.

In einer vorteilhaften Ausführungsform betrifft die vorliegende Erfindung daher auch ein Wasserfahrzeug wie hier beschrieben, bestehend aus einem Verbund von zwei oder mehr einzelnen Wasserfahrzeugen, dadurch gekennzeichnet, dass ein erstes Wasserfahrzeug die Anlagen a) - e) enthält und die damit verbundenen weiteren Wasserfahrzeuge jeweils eine Speicheranlage f) enthalten. In dieser Ausführungsform wird sowohl die Betriebssicherheit erhöht als auch die Flexiblität der gesamten Anlage verbessert.

In einer weiteren vorteilhaften Ausführung ist die Methanol- Speicheranlage mit einer beweglichen Trennwand (z.B. einer Membran) in zwei Teilvolumina getrennt. So ist es möglich, in der Anlage Wasser zu speichern, welches im Laufe der Reaktion benötigt wird. Das Volumen des verbrauchten Wassers entspricht in etwa dem Volumen des gebildeten Methanols. Durch diese Massnahme ist es möglich, auf eine lokale Wasseraufabeitungsanlage zu verzichten. Ferner führt bei geeigneter Anordnung der Speicheranlage auf einem Schwimmkörper diese Ausführungsform zu einer erhöhten Stabilität.

**Nebenanlagen:** Neben diesen notwendigen Anlagen kann der Schwimmkörper noch weitere Anlagen ("Nebenanlagen" bzw. Module) enthalten. Dies umfasst solche Anlagen, die zur Reinigung von Ausgangsmaterialien, zur Bereitstellung und Rückgewinnung von Hilfsstoffen und zur Rückgewinnung von Energie, insbesondere thermischer Energie, geeignet sind. Ferner können Zwischenspeicher für die intermediär entstehenden Produkte vorgesehen werden, so z.B. Batterien für die Zwischenspeicherung elektrischer Energie, Gastanks für die Zwischenspeicherung von CO2, und H2. Flüssigtanks für die Zwischenlagerung von Hilfsstoffen. Ebenfalls können Anlagen zur Regenerierung der Katalysatoren und der Elektrolysezellen vorgesehen werden. Ferner können Speicher für den ggf. erzeugten Sauerstoff vorgesehen werden. Diese Nebenanlagen sind an sich bekannt und können vom Fachmann entsprechend ausgelegt werden.

**Skalierung:** Die Skalierung eines erfindungsgemässen Kraftwerkes hängt von verschiedenen Parametern ab und ist durch die Erfindung nicht begrenzt. Typischerweise stellt die zur Verfügung stehende Wind- oder Sonnenenergie und die dafür bekannten Anlagen eine obere Begrenzung dar. Zur Charakterisierung der Grösse des erfindungsgemässen Kraftwerkes eignet sich die Angabe der primär erzeugten elektrischen Energie (durch Wind- oder Solarenergie), da die weiteren Anlagen darauf abgestimmt werden. So wäre bei Grossanlagen eine einzelne Windkraftanlage mit ca. 10 MW als obere Leistungsgrenze zu nennen. Auf diese Leistung sind die weiteren Komponenten auszulegen. Typischerweise stellt die Grösse der Anlagen d) bis e) eine untere Begrenzung dar, da unterhalb einer kritischen Grösse die Anlagen für eine effiziente Arbeitsweise zu klein sind. In Verbindung mit einer Direkt Methanol Brennstoffzelle wären ca. 100 W als untere Leistungsgrenze zu nennen. Auf diese Leistung sind entsprechend die weiteren Anlagen auszulegen. Somit betrifft die Erfindung ein netzungebundenes Kraftwerk mit einer Leistung von 100 W bis 10 MW, bevorzugt von 1 kW bis 5 MW Leistung, besonders bevorzugt von 0.5 bis 3 MW Leistung.

Die Erfindung betrifft insbesondere auch ein Kraftwerk welches bevorzugt an Orten mit konstanten Windverhältnissen bzw. geeigneten Strömungen des Wassers oder geeigneten Wellenbewegungen des Wassers (wie nachstehend beschrieben) positioniert ist. Diese Positionierung löst viele der Probleme bestehender Windkraftanlagen (bei landgebundenen Anlagen: insbesondere Lärm, Landschaftsbild), bei Offshore Anlagen: insbesondere Kosten für Fundamente, Wartung und Netzwerkanbindung) bzw. Wasserkraftanlagen.

Für unverankerte Offshore - Anlagen besteht somit erstmals die Möglichkeit, einen Energieträger zu erzeugen, der (i) konzentriert gespeichert; (ii) gut transportiert und (iii) in bestehender Infrastruktur eingesetzt werden kann. Zum Vergleich sei darauf verwiesen, dass für Wasserstoff als Energieträger weder eine bestehende Infrastruktur vorliegt, ferner Speicherung und Transport mit erheblichen technischen Problemen verbunden sind.

In einem **zweiten Aspekt** betrifft die vorliegende Erfindung einen Schwimmkörper, insbesondere ein Wasserfahrzeug, umfassend die folgenden Anlagen: a) Windkraftanlage oder Wasserkraftanlage zur Erzeugung elektrischer Energie für den Betrieb der Anlagen b) bis f); b) CO2 - Absorptionsanlage zur Absorption von atmosphärischem CO2; c) CO2 - Desorptionsanlage zur Desorption des in b)gewonnenen CO2: d) elektrochemische H2 - Syntheseanlage; e) katalytische Syntheseanlage zur Erzeugung brennbarer Kohlenwasserstoffe; f) Speicheranlage zur Speicherung der in e) gewonnenen Kohlenwasserstoffe. Ein derartiger Schwimmkörper bietet die Möglichkeit, brennbare Kohlenwasserstoffe (wie Methan, DME oder Methanol) aus den praktisch unbegrenzten Ressourcen CO2 der Luft und H2 des Wassers mittels Windenergie oder Wasserkraft zu erzeugen und zu lagern. Da alle notwendigen Ressourcen kostenfrei zur Verfügung stehen ist es dadurch möglich, diese brennbaren Kohlenwasserstoffe als wertvollen und umweltneutralen Energieträger kostengünstig zu produzieren. Ferner ist die Lagerung solcher Kohlenwasserstoffe auf einer solchen Vorrichtung möglich. Die einzelnen Anlagenteile a) bis f) sind an sich bekannt und können auf Basis des allgemeinen Fachwissens dimensioniert und auf die Erfordernisse im Betrieb einer schwimmfähigen Vorrichtung angepasst werden. Bevorzugt werden die Anlagen a) bis f) so ausgestaltet wie in diesem Dokument beschrieben.

Ein solcher Schwimmkörper kann fern vom Verbraucher installiert werden, da weder Leistungsverluste durch Übertragung noch Kosten für die Netzwerkanbindung entstehen, was z.B. einen interkontinentalen Transport von Energie auch unter wirtschaftlichen Gesichtspunkten ermöglicht. Ein Grundgedanke der vorliegenden Erfindung ist daher, regenerative Energie dort zu "ernten" wo sie gleichmässig und in hoher Dichte anfällt, diese gewonnene Energie in einen chemischen Energieträger zu überführen welcher gespeichert und diskontinuierlich zur weiteren Verwendung entleert wird.

Ferner kann ein solcher Schwimmkörper leicht repositioniert werden; bspw. um Wartungsarbeiten durchzuführen oder um in Regionen mit optimalen Wind- / Wellen- / Strömungs- Verhältnissen zu operieren.

In einer Ausführungsform betrifft die Erfindung daher ein Wasserfahrzeug als Schwimmkörper, welches mit einer WKA ausgerüstet ist. In dieser Ausführungsform kann der axiale Widerstand des Rotors für den Vortrieb des Schwimmkörpers benutzt werden, um so die Positionierung / Re-Positionierung zu ermöglichen oder zu unterstützen.

In einer weiteren Ausführungsform betrifft die Erfindung daher eine Boje als Schwimmkörper, welche mit einer WKA ausgerüstet ist.

In einer weiteren Ausführungsform betrifft die Erfindung daher ein Segelboot als Schwimmkörper, welches mit einer Wasserturbine ausgerüstet ist.

In einem **dritten Aspekt** betrifft die vorliegende Erfindung die Verwendung eines netzungebundenen Kraftwerkes bzw. eines Schwimmkörpers wie hier beschrieben, insbesondere einer Boje oder eines Wasserfahrzeuges, zur Erzeugung und Speicherung von brennbaren Kohlenwasserstoffen wie Methanol Methan, DME,. Das Kraftwerk ist so ausgelegt, das der wesentliche oder ausschliessliche Zweck die Produktion und das (Zwischen)speichern des brennbaren Kohlenwassserstoffes ist. Der Schwimmkörper kann so ausgelegt sein, dass sein wesentlicher oder ausschliesslicher Bestimmungszweck die Produktion und das (Zwischen-) Speicherung von brennbarem Kohlenwasserstoff ist; ebenso ist es möglich, das diese Anlagen als Hilfsaggregate zum Antrieb des Schwimmkörpers verwendet werden oder das der erzeugte Kohlenwasserstoff in weiteren Prozessen verwendet wird. Bevorzugt ist es, den erfindungsgemässen Schwimmkörper zur Produktion und Lagerung von brennbaren Kohlenwasserstoffen, insbesondere Methan oder Methanol oder DME, zu verwenden.

In einem **vierten Aspekt** betrifft die Erfindung ein Verfahren zur Herstellung von brennbaren Kohlenwasserstoffen (insbesondere Methanol, Methan und/oder DME) umfassend die Schritte a) Positionieren eines Kraftwerkes bzw. eines Schwimmkörpers wie hier beschrieben in einer Region welche konstante Windverhältnisse, konstante Wellenbewegung oder konstante Sonneneinstrahlung aufweist; b) Betrieb der Anlagen dieses Kraftwerkes / Schwimmkörpers; c) diskontinuierliche, bevorzugt periodische, Entleerung der Speicheranlage. Dieses Verfahren ermöglicht es, in einfacher und sicherer Weise einen chemischen Energieträger zu produzieren sowie flexibel und bedarfsgerecht zur Verfügung zu stellen. Die einzelnen Schritte werden nachfolgend erläutert.

**Schritt a)** Regionen mit konstanten Windverhältnissen sind allgemein bekannt; Die notwendige Stärke des Windes hängt von der Auslegung der Windkraftanlage ab. Typischerweise sollte eine mittlere Windgeschwindigkeit von mindestens 7m/s vorliegen. Konstant werden Windverhältnisse dann betrachtet, wenn die Mittlere Windgeschwindigkeit zu mindestens 70%, bevorzugt zu mindestens 80% der Zeit vorliegt. Typische Regionen sind die Gebiete des Passatwindes und Gebiete, welche eine "natürliche Düse" bilden. (wie küstennahe Gebiete, Gebiete zwischen Inseln, an Gebirgspässen, auf Gipfeln usw.) Generell ist bei der Positionierung zu berücksichtigen, dass konstanter Wind den Nutzungsgrad erhöht; starker Wind eine hohe Generatorleistung bei kleiner Rotorfläche ermöglicht; wenig vertikale Windscherung den Verschleiss reduziert und ebenfalls die Leistung erhöht. Insbesondere Passatwinde erfüllen diese Erfordernisse, was einen erheblichen Vorteil gegenüber landgebundenen Anlagen darstellt.

Regionen mit konstanten Wellenbewegungen sind allgemein bekannt; Die notwendige Stärke der Wellen hängt von der Auslegung der Wellenkraftanlage ab. Typischerweise sollten Wellen mit einer Höhe von <10 m, bspw. 2 - 8 m vorliegen. Konstant werden Wellenverhältnisse dann betrachtet, wenn die Wellenhöhe im Jahresdurchschnitt mehr als 0.7 m beträgt. Typische Regionen sind die Gebiete des Südpazifik.

Regionen mit konstanter Sonneneinstrahlung sind ebenfalls allgemein bekannt. Als geeignet sind Gebiete anzusehen, in denen die durchschnittliche Intensität der Sonneneinstrahlung im Jahresschnitt mehr als 200 Watt / m2 beträt. Typische Gebiete sind bspw. auf der Arabischen Halbinsel oder im nördlichen Afrika.

Die Positionierung des Wasserfahrzeuges kann auf verschiedene Arten erfolgen. Im einfachsten Fall wird das Wasserfahrzeug an der gewünschten Position verankert. Alternativ kann das Wasserfahrzeug im Wind kreuzen, wobei es, je nach Konstruktion, durch Motorkraft und/oder Windkraft auf Kurs gehalten wird. In einer weiteren Alternative wird das Wasserfahrzeug in bestimmten Zeitabständen re-positioniert, bspw. bei sich ändernder Wetterlage oder aufgrund jahreszeitlicher Schwankungen. Die Erfindung betrifft daher auch ein Verfahren, bei dem das Positionieren des Schwimmkörpers gegebenenfalls ein kontinuierliches oder diskontinuierliches Repositionieren mit umfasst.

In einer Vorteilhaften Ausführungsform kann der Widerstand der Windkraftanlage zum Antrieb des Schwimmkörpers, insbesondere eines Schiffes, teilweise oder vollständig genutzt werden. Die Erfindung betrifft daher auch ein Verfahren wie hier beschrieben, in welchem die Positionierung und/oder Re-Positionierung der Schwimmfähigen Vorrichtung unter teilweiser oder vollständiger Ausnutzung des Widerstandes der Windkraftanlage erfolgt.

Die Positionierung eines Kraftwerkes welches nicht auf einem Schwimmkörper installiert ist betrifft im Wesentlichen die vorteilhafte Aufstellung der Windkraftanlage bzw. Solarthermischen Anlage, wobei auch hier die genannten Kriterien anwendbar sind.

**Schritt b)** Der Betrieb der einzelnen Anlagen erfolgt entsprechend der Auslegung auf bekannte Art und weise. Die einzelnen Anlagenteile müssen in Ihrer Produktionskapazität aufeinander abgestimmt werden. Beispielsweise muss die erzeugte elektrische Energie für alle Anlagenteile b) bis f) sowie gegebenenfalls vorhandene Nebenaggregate ausreichen, ferner muss die Produktion von H2 und die Desorption von CO2 aufeinander abgestimmt werden. Ferner können die Anlagen so ausgelegt sein, dass eine vollautomatische, fernüberwachte Produktion möglich ist oder dass eine Bedienmannschaft vor Ort ist, sowie Kombinationen daraus. Sofern das Wasserfahrzeug vor Anker liegt, ist ein vollautomatisierter Betrieb möglich. Sofern das Wasserfahrzeug im Wind kreuzt, ist zumindest ein teilweise manueller Betrieb bevorzugt, der zumindest die Positionierung des Wasserfahrzeuges überwacht und regelt.

**Schritt c)** Die Entleerung der Speicheranlage erfolgt erfindungsgemäss diskontinuierlich, bevorzugt in periodischen Zeiträumen. Als Parameter dafür kann entweder eine Zeiteinheit verwendet werden (z.B. monatlich, quartalsweise, jährlich) oder der Füllstand der Speicheranlage (z.B. mindestens zu 50% gefüllt, maximal zu 90% gefüllt), oder ein räumlicher Parameter (z.B. indem ein Wasserfahrzeug beim Kreuzen im Passatwind einen bestimmten Kurs fährt), oder Kombination daraus. Im Zusammenhang mit der vorliegenden Erfindung ist der Begriff "periodisch" zumindest nicht im exakt mathematischen Zusammenhang zu verstehen; vielmehr wird der produzierte chemische Energieträger auf der schwimmfähigen Vorrichtung produziert und gelagert und diskontinuierlich an weitere Verbraucher abgegeben.

Die nachfolgenden **Ausführungsbeispiele** sollen die Erfindung weiter erläutern ohne sie zu beschränken.

### Bsp.1 Boje mit Rotor

Ein Schwimmkörper wird mit den folgenden Anlagen ausgerüstet:
■ ein 600 kW Windkraftgenerator als Modul a
■ ein Gebläse - Luftbefeuchter als Modul b; Querschnittsfläche = 9 m2; v = 3.5 m/s, offener Kontakt zur wässrigen NaOH-Lösung im Waschturm
■ ein Schwefelsäure enthaltender Regenerator zur CO2 Freisetzung mit anschliessender Elektrodialyse als Modul c;

   H2S04 + Na2C03 -> Na2S04 + CO2 + H2O

   Na2S04 + H2O -> NaOH + H2SO4.
■ eine Elektrolyse - Zelle zur Elektrolyse von Wasser als Modul d

   3 H2O -> 3H2 + 3/2 02; Kapazität: 84 kg H2O/h
■ eine Methanol Synthese Anlage mit Destillationseinheit als Modul e; Kapazität 50 kg Methanol/h.
■ einen Tank zur Aufnahme des gebildeten Methanols als Modul f, Volumen = 300m3.

Dieses Kraftwerk produziert 50 kg Methanol/h, was einem Energiegehalt Hu von 274 kWh entspricht (Wirkungsgrad 46 %). Die Komponenten Natronlauge und Schwefelsäure werden recycliert; CO2 und H2O werden der Umgebung entnommen.

### Bsp. 2 Windkraftwerk

Ein Kraftwerk mit folgenden Anlagen
■ eine horizontale Windkraftanalge mit Getriebe, deren Rotorblätter mit einer HF Membran beschichtet sind als kombinierte Modul a und b.
■ eine Schwefelsäuredesorptinosanlage mit nachgeschalteter Elektrodialyse als Modul c
■ eine PEM Elektrolyseanlage als Modul d)
■ eine Methansyntheseanalge ohne Destillationseinheit als Modul e
■ ein CNG Tank als Modul f)

Ein solches Kraftwerk kann bspw. auf einer Insel im Nordatlantik installiert werden.

### Bsp. 3 Segelschiff

Ein Segelschiff wird mit folgenden Anlagen ausgerüstet
■ eine am Rumpf montierte, im Wasser liegende Turbine als Modul a
■ ein CO2 Absorptionsanlage mit Verdunstungsinhibitoren Prinzip Offener Kontakt im Gegenstrom mit Füllkörpern als Modul b
■ eine Dreikammerelektrolyse als kombiniertes Modul c) und d)
■ eine Methanolsyntheseanalge mit Destillationseinheit als Modul e
■ ein Methanoltank als Modul f)

Ein solches Schiff kann bspw. im Passatwind kreuzend betrieben werden.

### Bsp. 4 rein thermisches Kraftwerk

Ein Kraftwerk mit den folgenden Modulen
■ eine trockene CO2 Absorptionsanlage, welche mittels CaO Nanopartikeln arbeitet als Modul b)
■ eine daran angepasste thermische CaCO3 Crack-Anlage mit H2 - Überlagerung als Modul c)
■ eine thermische H2 Syntheseanlage nach dem ZnO/H2O Zyklus als Modul d)
■ eine Methansyntheseanalge ohne Destillationseinheit als Modul e
■ ein LNG Tank als Modul f)

Ein solches Kraftwerk kann bspw. auf der arabischen Halbinsel installiert werden. Als Modul a) wird ein Gasmotor vorgesehen, der das erzeugte Methan als Brennstoff verwendet. Ein Modul a ist in dieser Ausführungsform lediglich zur Versorgung von Hilfsaggregaten nötig.

### Bsp. 5 Wellenkraftwerk

Ein Schiff wird mit den nachstehend beschriebenen Modulen b) bis f) ausgerüstet, mehrere Module a) sind damit verbunden
■ eine Wellenkraftanlage vom Typ Pelami als Modul a)
■ eine nasschemische CO2 Absorptionsanlage, welche unbeschichtete HF Membranen nutzt als Modul b)
■ eine SOEC Anlage als kombiniertes Modul c) und d)
■ eine DME - Syntheseanalge ohne Destillationseinheit als Modul e)
■ ein LPG Tank als Modul f)

Ein solches Kraftwerk kann bspw. im Südpazifik installiert werden.

Der nachfolgende morphologische Kasten stellt die genannten 5 Beispiele sowie weitere Ausgestaltungen der Erfindung in übersichtlicher Weise zusammen, ohne dass er diese Erfindung beschränkt.

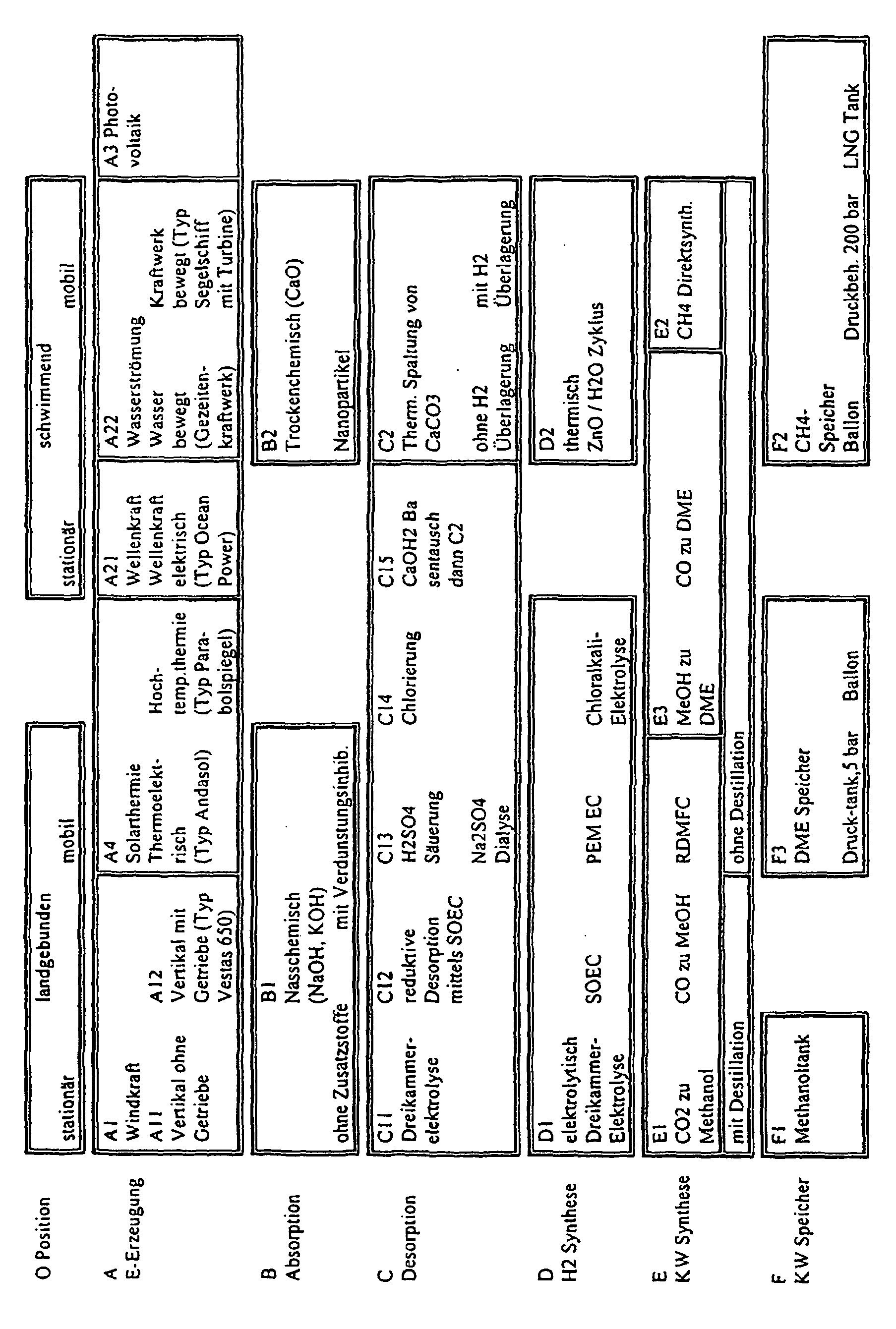

## Patentansprüche

1. Netzungebundenes Kraftwerk, **dadurch gekennzeichnet, dass** es auf einem Schwimmkörper installiert ist und dass es die folgenden, in Ihrer Kapazität aufeinander abgestimmten Anlagen enthält:
a) Anlage zur Erzeugung elektrischer und / oder thermischer Energie (A), ausgewählt aus der Gruppe umfassend Windkraftanlage, Wasserkraftanlage, Photovoltaikanlage und /oder solarthermische Anlage, zum Betrieb der Anlagen b) bis f);
b) CO2 - Absorptionsanlage (B) zur Absorption von atmosphärischem CO2;
c) CO2 - Desorptionsanlage (C) zur Desorption des in b)gewonnenen CO2;
d) elektrochemische oder solarthermische H2 - Syntheseanlage (D) zum Betrieb der Anlage e);
e) Syntheseanlage (E) ausgewählt aus der Gruppe katalytische Methanol - Syntheseanlage; katalytische Dimethylether - Syntheseanlage, katalytische Methan - Syntheseanlage;
f) Speicheranlage (F) ausgewählt aus der Gruppe Methanol - Speicheranlage, Dimethylether - Speicheranlage, Methan - Speicheranlage.

2. Netzungebundes Kraftwerk gemäss Anspruch 1, **dadurch gekennzeichnet, dass** die Anlage zur Erzeugung elektrischer Energie (A) eine Windkraftanlage ist.

3. Netzungebundes Kraftwerk gemäss Anspruch 1, **dadurch gekennzeichnet, dass** die Anlage zur Erzeugung elektrischer Energie (A) eine Wasserkraftanlage, insbesondere eine an einem Schwimmkörper befestige Wellenkraftmaschine oder Turbine ist.

4. Netzungebundes Kraftwerk gemäss einem der vorstehenden Ansprüche, **dadurch gekennzeichnet, dass** in der CO2-Absorptionsanlage der Luftstrom ggf. zunächst befeuchtet wird und dann mit alkalischen Waschlauge im Gegenstrom in Kontakt gebracht wird.

5. Netzungebundes Kraftwerk gemäss einem der vorstehenden Ansprüche, **dadurch gekennzeichnet, dass** die Rotorblätter und / oder der Mast der Windkraftanlage a) mit hydrophoben mikroporösen Membranen zur CO2 Absorption versehen sind, insbesondere wobei besagte hydrophoben mikroporösen Membranen mit hygroskopischen Material, belegt sind.

6. Netzungebundes Kraftwerk gemäss einem der vorstehenden Ansprüche, **dadurch gekennzeichnet, dass** die CO2-Desorptionsanlage b) nach dem Prinzip der Säuerung mit ggf. anschliessender Elektrodialyse arbeitet.

7. Netzungebundes Kraftwerk gemäss einem der vorstehenden Ansprüche, **dadurch gekennzeichnet, dass** die elektrochemische H2 - Syntheseanlage (D) nach dem Prinzip der Hochtemperatur-Elektrolyse von Wasser, welches ggf. entsalzt ist, arbeitet.

8. Netzungebundes Kraftwerk gemäss einem der vorstehenden Ansprüche, **dadurch gekennzeichnet, dass** die katalytische Syntheseanlage (E) entweder
a) eine Methanol - Syntheseanlge ist, welche einen Cu-haltigen Katalysator enthält, und das dieser Anlage ggf. eine Destillationseinheit zugeordnet ist oder
b) eine Methan - Syntheseanlage ist, welche einen Ni-haltigen Katalysator enthält, und das dieser Anlage ggf. keine Destillationseinheit zugeordnet ist.

9. Netzungebundes Kraftwerk gemäss einem der vorstehenden Ansprüche, **dadurch gekennzeichnet, dass** die CO2-Desorptionsanlage c) unter reduktiven Bedingungen aus eingesetzten Carbonat CO erzeugt und die Methanol-Syntheseanlage e) eine Methanol / Synthesegas-Anlage ist.

10. Netzungebundes Kraftwerk gemäss einem der vorstehenden Ansprüche, **dadurch gekennzeichnet, dass** die CO2-Absorptionsanlage (B) ein Nasschemische CO2-Absorptionsanlage enthaltend eine Waschlösung ist, und wobei die Waschlösung bevorzugt eine die Verdunstung reduzierende Komponente enthält.

11. Schwimmkörper, insbesondere Boje oder Schiff, enthaltend ein Kraftwerk gemäss einem der Ansprüche 1 - 10.

12. Schwimmkörper gemäss Anspruch 11 **dadurch gekennzeichnet, dass** es aus einem Verbund von zwei oder mehr einzelnen Schwimmkörpern besteht wobei
a) eine erste Gruppe von Schwimmkörpern die Module a) - e) enthält und eine damit verbundene zweite Gruppe von Schwimmkörpern Speicheranlage(n) f) enthalten oder
b) eine erste Gruppe von Schwimmkörpern Modul(e) a), insbesondere Wellenkraftmaschinen, enthält und eine damit verbundene zweite Gruppe von Schwimmkörpern Module b) - f) enthält.

13. Verwendung eines netzungebundenen Kraftwerkes gemäss einem der Ansprüche 1 bis 10 oder eines Schwimmkörpers gemäss einem der Ansprüche 11 - 12, zur Herstellung und Speicherung von Methanol, Dimethylether und/oder Methan.

14. Verfahren zur Herstellung von Methanol, Dimethylether und/oder Methan umfassend die Schritte
a) Positionieren eines netzungebundenen Kraftwerkes gemäss einem der Ansprüche 1 bis 10 oder eines Schwimmkörpers gemäss einem der Ansprüche -11 - 12 in einer Region mit konstanten Windverhältnissen und / oder konstanter Sonneneinstrahlung und/oder konstanter Wasserbewegung;
b) Betrieb der Anlagen dieses Kraftwerkes;
c) diskontinuierliche Entleerung der Speicheranlage.

## Claims

1. Power Plant, unlinked to a grid, **characterized in that** it is installed on a Floating Device and **in that** it comprises the following Modules matching with each other:
a) Module to produce electric and / or thermal power (A), selected from the group consisting of Wind Power Station, Water Power Station, Photovoltaic Power Station and /or Solar Thermal Power Station, to operate Modules b) to f);
b) Module for CO2 - Absorption (B), to absorb atmospheric CO2;
c) Module for CO2 - Desorption (C), to desorb CO2 obtained in b);
d) Module for electrochemical or solar thermal H2 - Synthesis (D), to operate Module e);
e) Synthesis Module (E) selected from the group consisting of Module for catalytic synthesis of methanol, Module for catalytic synthesis of dimethyl ether, Module for catalytic synthesis of methane;
f) Storage Module (F) selected from the group consisting of Storage Module for methanol, Storage Module for dimethyl ether, Storage Module for methane.

2. Power Plant, unlinked to a grid, according to claim 1, **characterized in that** said Module (A) for producing electric power is a Wind Power Station.

3. Power Plant, unlinked to a grid, according to claim 1, **characterized in that** said Module (A) for producing electric power is a Water Power Station, particularly a wave power machine or a turbine attached to a Floating Device.

4. Power Plant, unlinked to a grid, according to any of the previous claims, **characterized in that** the airflow in said Module for CO2 - Desorption (B) is optionally humidified at first, and contacted with an alkaline suds by way of counter flow afterwards.

5. Power Plant, unlinked to a grid, according to any of the previous claims, **characterized in that** the rotor blades and / or the mast of said Wind Power Station a) is equipped with hydrophobic micro-porous membranes for CO2 absorption, particularly whereby said hydrophobic micro-porous membranes are coated with hygroscopic material.

6. Power Plant, unlinked to a grid, according to any of the previous claims, **characterized in that** said Module for CO2 - Absorption b) operates according to the principle of acidification, optionally followed by electrodialysis.

7. Power Plant, unlinked to a grid, according to any of the previous claims, **characterized in that** the Module for electrochemical H2 - Synthesis d) operates according to the principle of high-temperature-electrolysis of water, which is optionally desalinated.

8. Power Plant, unlinked to a grid, according to any of the previous claims, **characterized in that** the catalytic Synthesis Module e) is
a) a Module for synthesis of methanol, which contains a Cu-type catalyst, and to which optionally a distillation unit is allocated;
or
b) a Module for synthesis of methane, which contains a Ni-type catalyst, and to which optionally no distillation unit is allocated.

9. Power Plant, unlinked to a grid, according to any of the previous claims, **characterized in that** the Module for CO2 - Desorption c) produces CO under reductive conditions from the carbonate used, and said Module for synthesis of methanol e) is a methanol / Syngas module.

10. Power Plant, unlinked to a grid, according to any of the previous claims, **characterized in that** the Module for CO2 - Absorption (B) is a wet-chemical CO2-Module for absorption comprising a suds, and whereby said suds preferably comprises a component that reduces evaporation.

11. Floating device, particularly buoy or vessel, comprising a Power Plant according to any of claims 1 - 10.

12. Floating device according to claim 11, **characterized in that** it comprises an assembly of two or more individual Floating Devices, whereby
a) a first group of Floating Devices comprises Modules a) - e) and a second group of floating devises, connected therewith, comprises Storage Module(s) (f); or
b) a first group of Floating Devices comprises Module a), particularly wave power machines, and a second group of floating devises, connected therewith, comprises Modules b) - (f).

13. Use of a Power Plant, unlinked to a grid, according to any of claims 1 to 10 or of a Floating Device according to any of claims 11 to 12, for manufacturing and storage of methanol, dimethyl ether and / or methane.

14. Method for manufacturing methanol, dimethyl ether and / or methane comprising the steps of
a) Positioning of a Power Plant, unlinked to a grid, according to any of the claims 1 to 10 or of a Floating Device according to any of claims 11 to 12 in a region of incessant wind regime and / or incessant solar irradiation and / or incessant water movement;
b) Operating the Modules of said Power Plant;
c) Discontinuous emptying of said Storage Module.

## Revendications

1. Centrale électrique qui n'est pas reliée au réseau et qui est **caractérisée en ce qu'**elle est installée sur un corps flottant et **en ce qu'**elle comporte les installations suivantes adaptées les unes aux autres dans leur capacité ;
a) installation (A) de production d'énergie électrique et/ou thermique sélectionnée dans le groupe comprenant des éoliennes, des installations de force motrice hydraulique, des installations photovoltaïques et/ou des installations de thermique solaire pour le fonctionnement des installations b) à f) ;
b) installation (B) d'absorption de CO2 pour l'absorption du CO2 atmosphérique ;
c) installation (C) de désorption de CO2 pour la désorption du CO2 obtenu dans b) ;
d) installation (D) de synthèse de H2 électrochimique ou de thermique solaire pour le fonctionnement de l'installation e)
e) installation (E) de synthèse choisie dans le groupe installation de synthèse catalytique du méthanol, installation de synthèse catalytique de l'oxyde de diméthyle, installation de synthèse catalytique du méthane ;
f) installation (F) de stockage choisie dans le groupe installation de stockage du méthanol, installation de stockage de l'oxyde de diméthyle, installation de stockage du méthane.

2. Centrale électrique qui n'est pas reliée au réseau suivant la revendication 1, **caractérisée en ce que** l'installation de production d'énergie électrique (A) est une installation de force motrice hydraulique, notamment une machine motrice ou une turbine fixée à un corps flottant.

3. Centrale électrique qui n'est pas reliée au réseau suivant la revendication 1, **caractérisée en ce que** l'installation (A) de production d'énergie électrique est une installation de force motrice hydraulique, notamment une machine utilisant la force des vagues ou une turbine fixée sur un corps flottant.

4. Centrale électrique qui n'est pas reliée au réseau suivant l'une des revendications précédentes, **caractérisée en ce que**, dans l'installation d'absorption de CO2, le courant d'air est d'abord le cas échéant humidifié et est ensuite mis en contact à contre courant avec une lessive alcaline de lavage.

5. Centrale électrique qui n'est pas reliée au réseau suivant l'une des revendications précédentes, **caractérisée en ce que** les pales du rotor et/ou le mât de l'éolienne a) sont pour l'absorption de CO2 munis de membranes hydrophobes microporeuses, lesdites membranes hydrophobes microporeuses étant garnies notamment de matière hygroscopique.

6. Centrale électrique qui n'est pas reliée au réseau suivant l'une des revendications précédentes, **caractérisée en ce que** l'installation b) de désorption du CO2 fonctionne suivant le principe de l'acidulation, en ayant éventuellement une électrodialyse venant ensuite.

7. Centrale électrique qui n'est pas reliée au réseau suivant l'une des revendications précédentes, **caractérisée en ce que** l'installation c) de synthèse de H2 électrochimique fonctionne suivant le principe de l'électrolyse à haute température de l'eau, laquelle est le cas échéant déminéralisée.

8. Centrale électrique qui n'est pas reliée au réseau suivant l'une des revendications précédentes, **caractérisée en ce que** l'installation d) de synthèse catalytique est soit
a) une installation de synthèse du méthanol, qui contient un catalyseur contenant du Cu et à laquelle est associée, le cas échéant, une unité de distillation, soit
b) une installation de synthèse du méthane qui contient un catalyseur contenant du Ni et à laquelle n'est pas associée éventuellement d'unité de distillation.

9. Centrale électrique qui n'est pas reliée au réseau suivant l'une des revendications précédentes, **caractérisée en ce que** l'installation c) du désorption du CO2 produit du CO dans des conditions réductrices à partir du carbonate utilisé et l'installation e) de synthèse du méthanol est une installation de méthanol/gaz de synthèse.

10. Centrale électrique qui n'est pas reliée au réseau suivant l'une des revendications précédentes, **caractérisée en ce que** l'installation (B) d'absorption du CO2 est une installation d'absorption de CO2 en voie chimique humide, contenant une solution de lavage et la solution de lavage contient de préférence un constituant réduisant l'évaporation.

11. Corps flottant, notamment bouée ou navire, comprenant une centrale électrique suivant l'une des revendications 1 à 10.

12. Corps flottant suivant la revendication 11, **caractérisé en ce qu'**il est constitué d'une combinaison de deux ou plusieurs corps flottants individuels, dans lequel
a) un premier groupe de corps flottants comporte les modules a) à e) et un deuxième groupe de corps flottant ainsi relié contient la ou les installations f) de stockage ou
b) un premier groupe de corps flottant contient le ou les modules a), notamment des machines utilisant la force des vagues et un deuxième groupe ainsi relié de corps flottant contient les modules b) à f).

13. Utilisation d'une centrale électrique qui n'est pas reliée au réseau suivant l'une des revendications 1 à 10 ou d'un corps flottant suivant l'une des revendications 11 et 12 pour la production et le stockage de méthanol, d'oxyde de diméthyle et/ou de méthane.

14. Procédé de production de méthanol, d'oxyde de diméthyle et/ou de méthane comprenant les stades
a) on met en position une centrale électrique qui n'est pas reliée au réseau suivant l'une des revendications 1 à 10 ou un corps flottant suivant l'une des revendications 11 et 12, dans une région ayant des conditions de vent constantes et/ou un ensoleillement constant et/ou un mouvement des eaux constants ;
b) on fait fonctionner les installations de cette centrale électrique ;
c) on vide d'une manière discontinue l'installation de stockage.
